# EUROPEAN PATENT APPLICATION

(11) **EP 0 664 140 A1**
(43) Date of publication of application: **26.07.1995**
(21) Application number: 94203340.8
(22) Date of filing: 16.11.1994
(51) Int. Cl.: A61M 25/10

(54) **Catheter with balloon**

(30) Priority: 19.01.1994 NL 9400089
(71) Applicant: CORDIS EUROPA N.V., NL-9301 LJ Roden (NL)
(72) Inventor: Linnewiel, Remco, NL-9997 PN Zandeweer (NL); van Werven-Franssen, Gerda Hendrika Maria, NL-9302 EK Roden (NL)
(74) Representative: 't Jong, Bastiaan Jacobus

(57) **Abstract**

The invention relates to a catheter comprising a tube-like basic body with at least two lumens, connecting pieces for the lumens at a proximal end and an inflatable member connected to one of the lumens at the distal end. The basic body comprises a separate tube-like element received in a central channel of the latter and a first lumen which has been formed inside that tube-like element. A second lumen has been formed by the interspace between the tube-like element and the central channel.

## Description

The invention relates to a catheter with a balloon. Such a catheter comprises, in the usual manner, a tube-like basic body with at least two lumens. An inflatable member has been attached to the distal end which is connected to one of the lumens. The member can be inflated via this lumen. Via the other lumen one can inject for instance contrast medium, so that the area surrounding the inflatable member inside the body of the patient can be made visible in a catheterization laboratory in order to position it carefully and carry out the treatment. Another embodiment of such a catheter is a guiding catheter with an occlusion balloon. In that case the second lumen is used for advancing interventional catheters. By inflating the occlusion balloon the blood vessel will be occluded locally.

In order to allow the "inflation medium" to pass through it at a sufficient flow rate, the lumen used for inflating the balloon should be sufficiently large. The second lumen should in addition to this have a certain minimum diameter for the purpose of advancing interventional catheters for instance. Because of these requirements regarding the effective diameter of the lumens, limits are set to the minimum diameter of the basic body. In spite of this there is a need for catheters of the type mentioned with an ever decreasing outside diameter. There is in particular a need for guiding catheters with a small outside diameter and a large inside diameter.

The object of the invention is to provide a catheter of the type described in the preamble of which the basic body can be made to have a small diameter.

With the catheter as characterised in claim 1 this aim is achieved. The coaxial construction results in a very favourable relation between the effective diameter of the lumens and the overall diameter of the material. The interspace between the tube-like element and the central channel does not need to be larger than 0,1 mm. This is sufficient to inflate the balloon with a gas, in particular CO₂. By employing these measures, the catheter according to the invention can be a guiding catheter with occlusion balloon with a, in relation to the outer diameter, relatively large effective diameter of the guiding lumen.

A favourable construction is characterised in claim 3.

The lumen of the tube-like element forms because of this the normal continuous lumen of the basic body for guiding the interventional catheters and/or contrast medium. The connection with the lumen formed by the interspace can be made in a Y-piece. In that case this second lumen is connected with the inflatable member.

With the intended relatively small outside diameter and relatively large inside diameter of the continuous lumen of the catheter according to the invention, it could be difficult to make a good connection between the inflatable member and the lumen which is designed for inflating the latter. With the measure as set out in claim 4 however, the desired connection can be made in a very simple and reliable manner.

According to a very favourable further development, the measure as set out in claim 5 is employed. Because of this the balloon has a very small profile which is not larger than the outside diameter of the basic body. The balloon at the distal end of the catheter can therefore be introduced into the body making use of conventional equipment.

For a good connection of the inflatable member with the second lumen formed by the interspace between the tube-like element and the central channel, the measure as set out in claim 6 is preferably employed. Thus the opening can be made sufficiently large.

With the measure as set out in claim 7 it is ensured that the inflatable member lies in a suitable manner on a small diameter in the recess of the basic body and can also be fixed with its end-sections firmly inside it.

To prevent that the slotted opening should be compressed due to the fact that the inflatable member is prestressed, the measure as set out in claim 8 is preferably employed. At all times an open connection between the interspace and the space inside the inflatable member is maintained at the rectangular ends of the slot.

The invention will be explained in the following description with reference to the attached drawings.
Fig. 1 shows a catheter according to the invention in a perspective view.
Fig. 2 shows a catheter of fig. 1 in a partly broken away perspective view on a larger scale.
Fig. 3 represents the inflatable member of the catheter of fig. 1 and 2.
Fig. 4 shows the section of the catheter to which the inflatable member of fig. 3 is attached and
Fig. 5 shows the inflatable member in activated state.

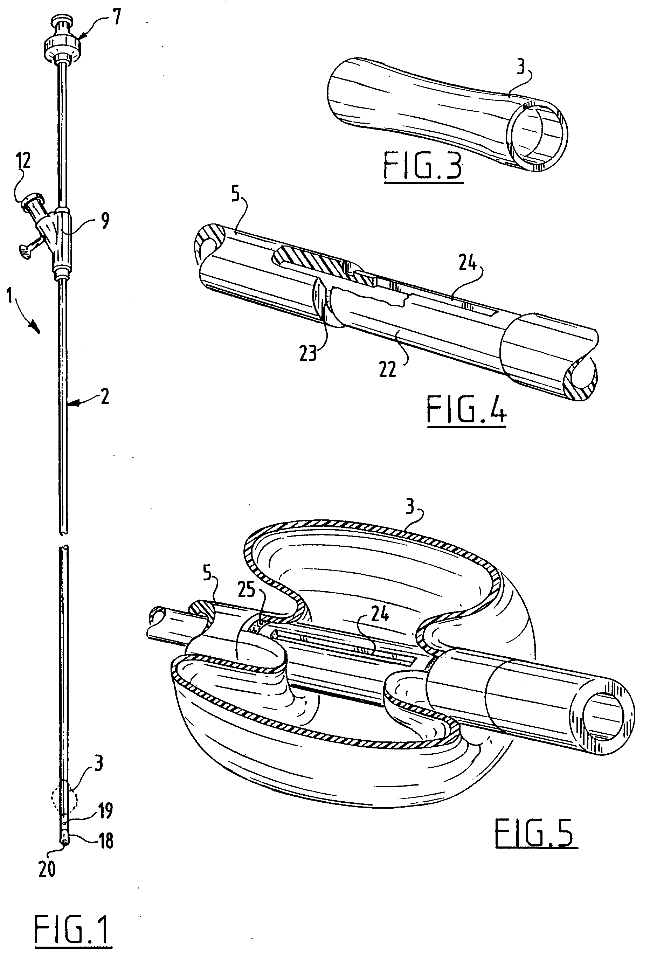

The guiding catheter 1 according to the invention as shown in fig. 1 comprises in the usual manner a tube-like basic body with at the distal end a balloon shaped member 3, to be explained in greater detail. There are two lumens in the basic body 2. One for inflating the balloon shaped body and the other for advancing an interventional catheter and, if necessary, contrast medium.

When treating a patient, the catheter according to the invention is introduced with the distal end close to the location where the treatment is to take place. One or more interventional catheters are advanced one after the other through the appropriate lumen to the site of the treatment. There the blood vessel can be occluded with the balloon.

In the central channel of the basic body 2 a separate tube-like element 6 has been received. Between the outer wall of this inner tube 6 and the wall of the central channel, an interspace 8 has been left. As will be explained in greater detail yet, the lumen of the inner tube forms the first lumen of the catheter 1 and the interspace 8 the second lumen of the catheter 1.

With a suitable embodiment, the outer tube-like section 5 of the basic body 2 has for instance an outside diameter of 3,0 mm and an inside diameter of 2,5 mm. The inner tube-like element 6 received inside it has for instance an outside diameter of 2,4 mm and an inside diameter of 2,05 mm. Consequently, there is an interspace of 0,1 mm diameter difference between the inner tube-like element and the outer tube-like element.

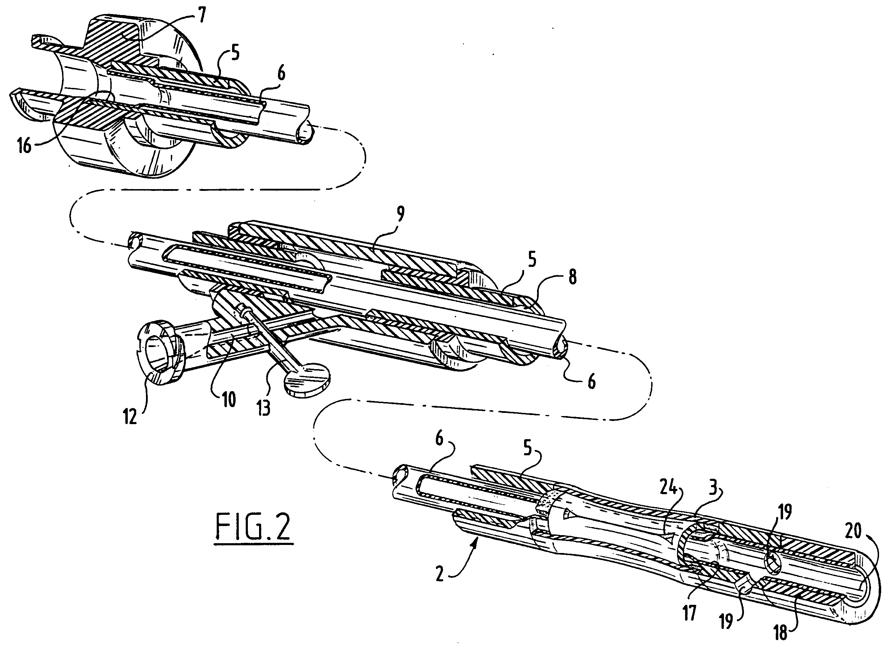

The inner tube-like element 6 is widened a little at the proximal end and is sealed all round to the inner wall of the central channel of the outer tube-like element 5. The widened section has been indicated with 16 in fig.2.

At the opposite distal end, a widened section 17 has been formed as well and the inner tube-like element is also sealed to the inner wall of the central channel of the outer tube 5.

The interspace 8 forming the second lumen is, at the position where a Y-piece 8 is arranged close to the proximal end, connected to a connecting member 12. As fig. 2 shows, the outer tube is interrupted at the Y-piece 9 and the ends are fastened in the Y-piece 9 in such a way as to form a seal, for instance by glueing. A channel 10 extends from the connecting member 12 to an internal cavity of the Y-piece 9, which is consequently connected with the interspace 8. The channel 10 can be closed by means of a rotatable valve member 13.

As mentioned before, the second lumen formed by the interspace 8 serves for supplying to and subsequently discharging "inflating medium" from the balloon-shaped element 3 which, in the example of the embodiment shown, is a gas, in particular CO².

The way in which the balloon-shaped element 3 has been received in the embodiment shown, will now be explained in greater detail with reference to the figures 3-5.

As fig. 3 shows, the balloon-shaped element 3 is in condition of rest a short tube section. The choice of material of which this tube section has been made is such, that it can be inflated to take on the shape as shown in fig. 5 without suffering permanent distortion. A suitable material is latex.

In the outer tube-like element 5 of the basic body 2, a section 22 with a reduced diameter has been formed as shown in fig. 4. This section 22 is obtained by a grinding operation.

At the ends of the section 22 with the reduced diameter also a groove 23 has been formed. In the wall of the section 22 with the reduced diameter a slot 24 has been made, which forms the connection between the interspace 8 and the inside of the balloon 3.

Attaching the tube section 3 to the section of the outer tube-like element prepared as described, occurs as follows. The tube section 3 is stretched and pushed over the catheter into the section 22. Then the ends of the tube section are turned back and glue 25 is applied to the grooves 23. Next the ends are rolled back again and are brought into contact with the glue. With the preferred embodiment as shown, the diameter of the section 22 has been reduced by the same amount as the wall thickness of the tube section 3, so that the outside surface of the tube section 3 merges smoothly into the outside surface of the tube-like element 5.

The balloon-shaped member 3 can be inflated by supplying medium under pressure to it via the connecting piece 12. This medium under pressure which, in the example in question is preferably CO₂, is brought via the interspace 8 and through the gap 24 to underneath the balloon 3, thus inflating the latter.

Fig. 5 shows the balloon 3 in inflated state.

Fig. 2 shows the balloon 3 in condition of rest. As the tube section 3 has been shrunk prestressed around the basic body in the way described, the slot 24 can be partly compressed as shown. As the slot has been made with rectangular ends however, at least triangular gaps, through which the air can be introduced into the balloon, will remain open at all times.

Fig. 2 also illustrates that at the free distal end the basic body is provided with an end section 18, which has been made of a soft plastic material.

As has been mentioned before, a liquid, in particular contrast medium, can be supplied to the catheter via the connecting piece 7. This medium will flow via the lumen of the inner tube-like element 6 to the distal end of the catheter. There the medium can flow out of the catheter either via the opening 20 in the tip of the catheter or via one or more holes 19 in the catheter wall.

## Claims

1. Catheter comprising a tube-like basic body with at least two lumens, connecting pieces for the lumens at a proximal end and an inflatable member connected to one of the lumens at the distal end, wherein the basic body comprises a separate tube-like element received in a central channel of the latter and a first lumen has been formed inside that tube-like element and a second lumen by the interspace between the tube-like element and the central channel, wherein the effective section of the first lumen is at least five times larger than that of the second lumen and the inflatable member is connected to the second lumen.

2. Catheter as claimed in claim, wherein the inflatable member is an occlusion balloon.

3. Catheter as claimed in claim 1 or 2, wherein the tube-like element is, close to the ends, sealed all round to the inner wall of the central channel.

4. Catheter as claimed in claim 3, wherein the inflatable member has been arranged around the basic body and the connection with the second lumen is formed by an opening in the wall of the basic body.

5. Catheter as claimed in claim 4, wherein the inflatable member in condition of rest is a tube-like body and has been received in a section of the basic body of which the diameter has been reduced to such a degree that the outer surface of the inflatable member in the condition of rest lies essentially in the same plane as the outer surface of the basic body.

6. Catheter as claimed in claim 4 and 5, wherein the hole in the wall of the basic body is a slot received in the section with a reduced diameter.

7. Catheter as claimed in one of the claims 5-6, wherein the inflatable body clamps prestressed around the basic body.

8. Catheter as claimed in claim 6 and 7, wherein the slot has rectangular ends.
